# EUROPEAN PATENT SPECIFICATION

(11) **EP 3 883 479 B1**
(45) Date of publication and mention of the grant of the patent: **26.06.2024**
(21) Application number: 19817544.0
(22) Date of filing: 19.11.2019
(51) Int. Cl.: A61B 10/02

(54) **BIOPSY DEVICE WITH MANUAL FIRING MECHANISM**
BIOPSIEVORRICHTUNG MIT MANUELLEM AUSLÖSEMECHANISMUS
DISPOSITIF DE BIOPSIE AVEC MÉCANISME DE DÉCLENCHEMENT MANUEL

(30) Priority: 20.11.2018 US 201862769956 P
(43) Date of publication of application: 29.09.2021
(73) Proprietor: Devicor Medical Products, Inc., Cincinnati, OH 45241 (US)
(72) Inventor: REBELLINO, Justin, Cincinnati, OH 45209 (US); MCBREEN, David, C., Cincinnati, OH 45069 (US)
(74) Representative: Simmons & Simmons
(86) International application number: PCT/US2019/062161
(87) International publication number: WO 2020/106691

(56) References cited:
- US-A1- 2006 155 210
- US-A1- 2007 106 176
- US-A1- 2012 109 007
- US-A1- 2012 283 563
- US-A1- 2015 126 902

## Description

### BACKGROUND

Biopsy samples have been obtained in a variety of ways in various medical procedures using a variety of devices. Biopsy devices may be used under stereotactic guidance, ultrasound guidance, MRI guidance, PEM guidance, BSGI guidance, or otherwise. For instance, some biopsy devices may be fully operable by a user using a single hand, and with a single insertion, to capture one or more biopsy samples from a patient. In addition, some biopsy devices may be tethered to a vacuum module and/or control module, such as for communication of fluids (e.g., pressurized air, saline, atmospheric air, vacuum, etc.), for communication of power, and/or for communication of commands and the like. Other biopsy devices may be fully or at least partially operable without being tethered or otherwise connected with another device. Other biopsy devices may be fully or at least partially operable without being tethered or otherwise connected with another device.

Merely exemplary biopsy devices are disclosed in U.S. Pat. No. 5,526,822, entitled "Method and Apparatus for Automated Biopsy and Collection of Soft Tissue," issued Jun. 18, 1996; U.S. Pat. No. 6,086,544, entitled "Control Apparatus for an Automated Surgical Biopsy Device," issued Jul. 11, 2000; U.S. Pat. No. 6,626,849, entitled "MRI Compatible Surgical Biopsy Device," issued Sept. 30, 2003; U.S. Pat. No. 7,442,171, entitled "Remote Thumbwheel for a Surgical Biopsy Device," issued Oct. 28, 2008; U.S. Pat. No. 8,764,680, entitled "Handheld Biopsy Device with Needle Firing," issued Jul. 7, 2014; U.S. Pat. No. 9,345,457, entitled "Presentation of Biopsy Sample by Biopsy Device, issued May 24, 2016; U.S. Pub. No. 2006/0074345, entitled "Biopsy Apparatus and Method," published Apr. 6, 2006, now abandoned; U.S. Pub. No. 2009/0171242, entitled "Clutch and Valving System for Tetherless Biopsy Device," published Jul. 2, 2009; U.S. Pub. No. 2010/0152610, entitled "Hand Actuated Tetherless Biopsy Device with Pistol Grip," published Jun. 17, 2010; and U.S. Pub. No. 2012/0310110, entitled "Needle Assembly and Blade Assembly for Biopsy Device," published Dec. 6, 2012.

In some circumstances, it may be desirable to incorporate a manually actuated needle firing mechanism into a biopsy device to fire a needle of the biopsy device directly into a lesion. For instance, in some conventional non-firing biopsy devices, the needle is manually inserted into the patient and then positioned below or adjacent to the lesion for collection of tissue samples from the lesion itself and/or adjacent tissue. In such devices, it may be desirable to still use manual insertion, but to also fire the needle directly into the lesion rather than place it adjacent to the lesion.

While several systems and methods have been made and used for obtaining a biopsy sample, it is believed that no one prior to the inventors has made or used the invention described in the appended claims.

US2012/0283563 discloses a biopsy system with a needle and cutter for severing a tissue sample, and a tissue sensor to sense a tissue sample.

US2012/0109007 discloses a biopsy system with a needle and cutter for severing a tissue sample, and a needle rotation assembly.

US2007/0106176 discloses a biopsy device with a cutting element which has an inner cannula with a tissue receiving aperture and an inner cannula.

US2006/0155210 discloses a biopsy instrument with improved needle penetration for piercing dense tissue.

US2015/0126902 discloses a biopsy device with a cutter extending through a lateral aperture of the needle and vacuum & fluid pumps.

### BRIEF DESCRIPTION OF THE FIGURES

While the specification concludes with claims which particularly point out and distinctly claim the biopsy device, it is believed the present biopsy device will be better understood from the following description of certain examples taken in conjunction with the accompanying drawings, in which like reference numerals identify the same elements and in which:
FIG. 1 depicts a perspective view of an exemplary biopsy device;
FIG. 2 depicts a perspective view of the biopsy device of FIG. 1, showing a holster detached from a probe;
FIG. 3 depicts a schematic view of exemplary electrical and/or electromechanical components of the holster of FIG. 2;
FIG. 4 depicts an exploded perspective view of the probe of FIG. 2;
FIG. 5 depicts a perspective view of an exemplary needle assembly and associated components of the probe of FIG. 2;
FIG. 6 depicts a cross-sectional perspective view of a distal end of the needle assembly of FIG. 5, taken along line 6-6 of FIG. 5;
FIG. 7 depicts an exploded perspective view of the needle assembly of FIG. 5;
FIG. 8 depicts an exploded perspective view of an exemplary needle firing assembly associated with the needle assembly of FIG. 5;
FIG. 9 depicts a detailed perspective view of a cocking portion of the needle firing assembly of FIG. 8;
FIG. 10A depicts a perspective view of the biopsy device of FIG. 1, with the needle firing assembly being moved to a cocked position;
FIG. 10B depicts a perspective view of the biopsy device of FIG. 1 being inserted into a patient;
FIG. 10C depicts a perspective view of the biopsy device of FIG. 1, with the needle firing assembly being fired;
FIG. 10D depicts a perspective view of the biopsy device of FIG. 1, with the biopsy device being used to collect a tissue sample;
FIG. 11A depicts a side cut-away view of the biopsy device of FIG. 1, with the needle firing assembly in a pre-cocked position;
FIG. 11B depicts another side cut-away view of the biopsy device of FIG. 1, with the needle firing assembly in the cocked position;
FIG. 11C depict yet another side cut-away view of the biopsy device of FIG. 1, with the needle firing assembly being fired;
FIG. 12A depicts a side elevational view of an exemplary alternative needle firing assembly for use with the biopsy device of FIG. 1; and
FIG. 12B depicts another side elevational view of the needle firing assembly of FIG. 12A, with the needle firing assembly in a fired position.

### DETAILED DESCRIPTION

### I. Overview of Exemplary Biopsy Device

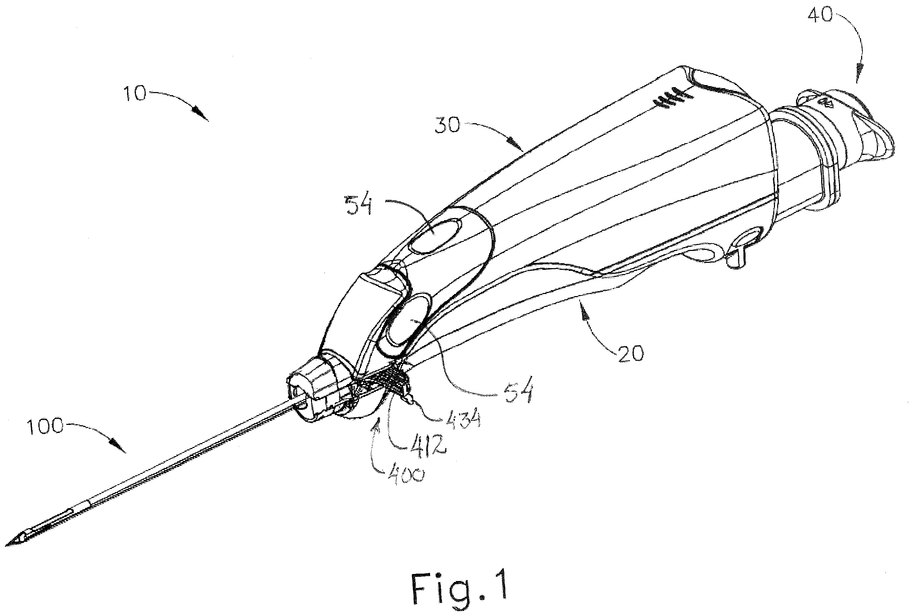

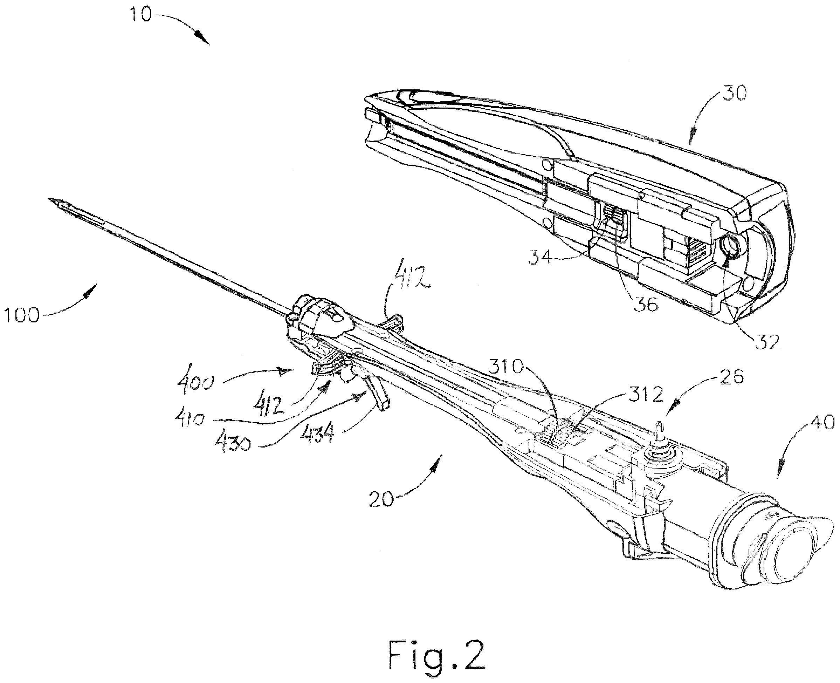

FIG. 1 shows an exemplary biopsy device (10), comprising a probe (20) and a holster (30). Probe (20) comprises a needle assembly (100) that at least partially extends distally from a casing of probe (20). Needle assembly (100) is insertable into a patient's tissue to obtain tissue samples as will be described below. Biopsy device (10) further comprises a tissue sample holder (40) into which the tissue samples are deposited. By way of example only, probe (20) may be a disposable component and holster (30) may be a reusable component to which probe (20) may be coupled, as is shown in FIG. 2. Use of the term "holster" herein should not be read as requiring any portion of probe (20) to be inserted into any portion of holster (30). Indeed, in one configuration for biopsy device (10), probe (20) may simply be positioned atop holster (30). Alternatively, a portion of probe (20) may be inserted into holster (30) to secure probe (20) to holster (30). In yet another configuration, a portion of holster (30) may be inserted into probe (20). Further still, probe (20) and holster (30) may be integrally formed as a single unit.

In configurations where probe (20) and holster (30) are separable members, a port and/or a seal (32) may be provided on holster (30) to couple with a second port and/or a seal (26) on probe (20) such that the vacuum produced by a vacuum pump (50) within holster (30) may be fluidly connected to probe (20). Holster (30) may also provide gears (34, 36) which mate to and engage with gears (310, 312) on probe (20). It should be understood that the configuration depicted in FIG. 2 that communicates vacuum and motive force between holster (30) and probe (20) is merely exemplary. In some versions, such configurations may be constructed in accordance with at least some of the teachings of U.S. Patent No. 8,206,316 entitled "Tetherless Biopsy Device with Reusable Portion," issued Jun. 26, 2012; and/or U.S. Pub. No. 2012/0065542, entitled "Biopsy Device Tissue Sample Holder with Removable Tray," published Mar. 15, 2012.

With holster (30) and probe (20) connected, vacuum pump (50) can induce a vacuum within needle assembly (100) via tissue sample holder (40) and a tubular cutter (60). However, it should be understood that vacuum may be provided in other ways. For example, vacuum pump (50) may be independent of holster (30) and probe (20) and may simply be coupled by vacuum tubes to appropriate ports on biopsy device (10). Biopsy device (10) may further be configured in accordance with at least some of the teachings of U.S. Patent No. 8,764,680, entitled "Handheld Biopsy Device with Needle Firing," issued July 1, 2014; and/or U.S. Pub. No. 2012/0065542, entitled "Biopsy Device Tissue Sample Holder with Removable Tray," published Mar. 15, 2012. Other suitable structural and functional combinations for probe (20) and holster (30) will be apparent to one of ordinary skill in the art in view of the teachings herein.

### II. Exemplary Holster

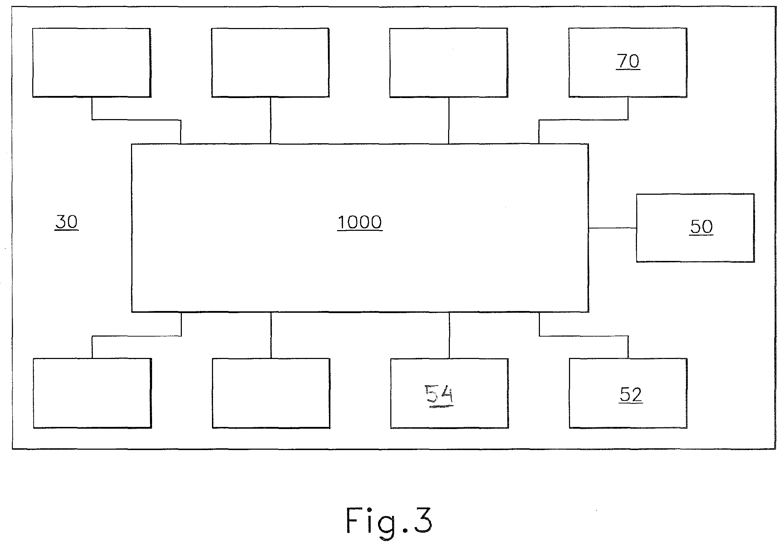

Holster (30), shown schematically in FIG. 3, comprises vacuum pump (50), a motor (70), a control module (1000), a plurality of buttons (54), a vacuum sensor (52), and any other suitable electrical and/or electromechanical components. Vacuum pump (50) of the present example comprises a conventional diaphragm pump that is mechanically coupled to motor (70). Vacuum sensor (52) is coupled to vacuum pump (50) or along any vacuum path therefrom such that vacuum sensor (52) can determine the level of vacuum created by vacuum pump (50). Vacuum sensor (52) is electrically coupled to control module (1000) so that vacuum sensor (52) may output signals indicative of the vacuum level to control module (1000). In the configuration shown, motor (70) is operable to translate and/or rotate cutter (60) in response to actuation of one or more of buttons (54), as will be described below, and to activate vacuum pump (50), though this is merely optional and a second motor (not shown) may be provided to run vacuum pump (50). In particular, motor may be coupled to a cutter drive assembly (not shown) and may be activated by control module (1000) upon actuating one or more of buttons (54). Such a cutter drive assembly (not shown) may rotate gears (34, 36) simultaneously. As noted above, gears (34, 36) mesh with gears (310, 312) in probe (20) thus allowing motor (70) to translate and/or rotate cutter (60). Other various configurations for holster (30) may be provided as will be apparent to one of ordinary skill in the art in view of the teachings herein. By way of example only, the cutter drive assembly (not shown) and/or other features of holster (30) may be constructed in accordance with at least some of the teachings of U.S. Patent 8,206,316 entitled "Tetherless Biopsy Device with Reusable Portion," issued Jun. 26, 2012; and/or U.S. Patent No. 8,764,680, entitled "Handheld Biopsy Device with Needle Firing," issued July 1, 2014.

### III. Exemplary Probe

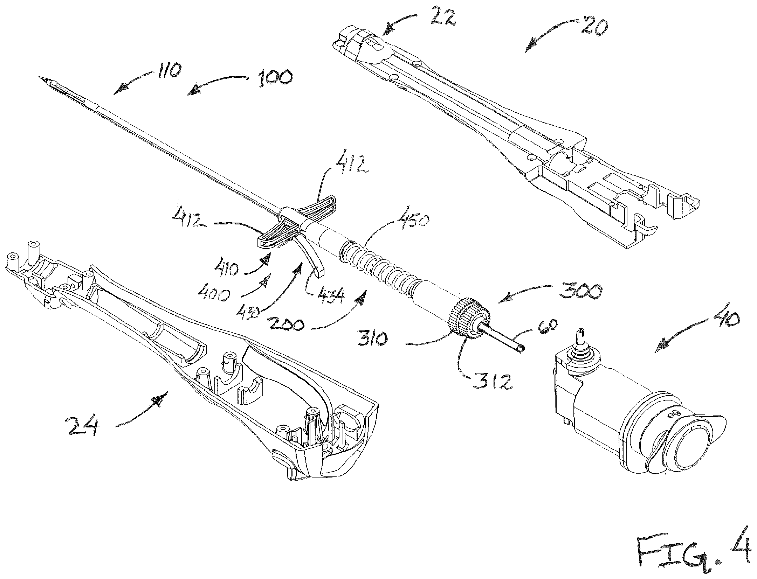

FIG. 4 depicts a partially exploded view of probe (20) showing needle assembly (100), a cutter actuation assembly (300), a probe housing (22, 24) and tissue sample holder (40). Needle assembly (100) comprises a needle portion (110) and a valve assembly (200). As will be described in greater detail below, needle assembly (100) is generally operable to pierce tissue where cutter (60) can be positioned to sever a tissue sample from a patient and transport the tissue sample to tissue sample holder (40). More specifically, the needle portion (110) of needle assembly (100) is inserted into a patient's tissue. Cutter actuation assembly (300) is then operable to selectively actuate cutter (60) to an open position after pressing one or more of buttons (54). Once cutter (60) is actuated by cutter actuation assembly (300) into an open position, tissue may be prolapsed into needle portion (110) by means of a vacuum communicated through cutter (60). Cutter (60) may then be selectively actuated by means of cutter actuation assembly (300) into the closed position, severing the prolapsed tissue from the patient. Vent assembly (300) is then operable to selectively vent a portion of needle portion (110) to atmosphere thus creating a pressure differential between proximal and distal ends of the prolapsed tissue. The pressure differential then transports the prolapsed tissue through cutter (60) to tissue sample holder (40).

### A. Exemplary Cutter Actuation Assembly

Cutter actuation assembly (300) comprises a series of gears (310, 312). Gears (310, 312) are configured to translate and/or rotate cutter (60). In the configuration shown, gears (310, 312) are coupled to motor (70) when probe (20) is attached to holster (30). In particular, two gears (310, 312) are controlled by motor (70) such that one gear (310) translates cutter (60) and another gear (312) rotates cutter (60) simultaneously. Other configurations may be provided utilizing different gear (310) arrangements. Moreover, configurations involving additional motors (70) may be used. Various suitable motor (70) and gear (310, 312) combinations will be apparent to one of ordinary skill in the art in view of the teachings herein. Indeed, cutter actuation assembly (300) may be constructed in accordance with at least some of the teachings of U.S. Patent 8,206,316, entitled "Tetherless Biopsy Device with Reusable Portion," issued Jun. 26, 2012.

### B. Exemplary Needle Portion

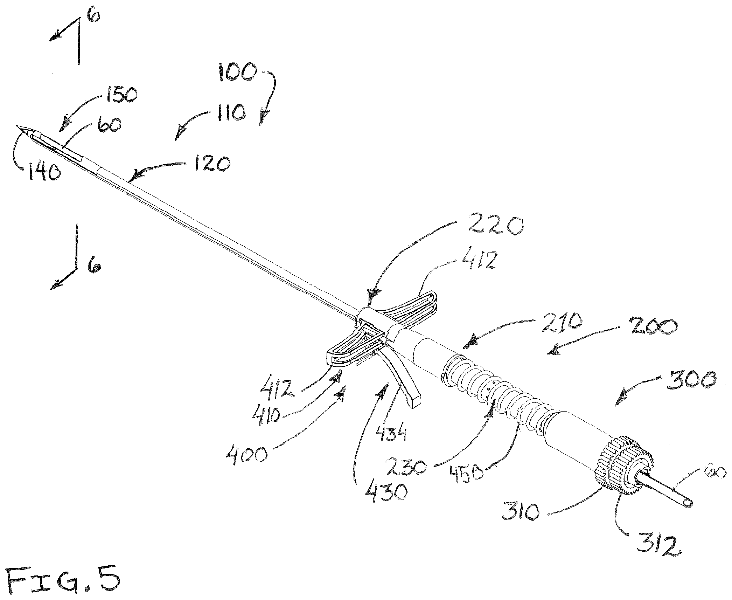

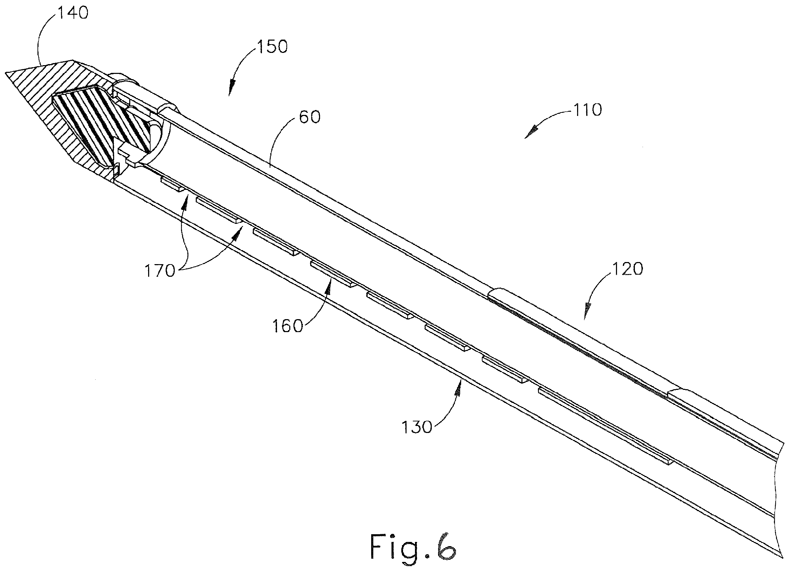

FIGS. 5 and 6 show an exemplary needle portion (110). Needle portion (110) comprises a cannula (120), a partial cannula (130), a tissue piercing tip (140), and a lateral aperture (150). As is shown, cannula (120) is positioned on top of partial cannula (130). Cannula (120) and partial cannula (130) define a first lumen portion (160) and a second lumen portion (162). As is best seen in FIG. 6, cannula (120) is generally circular in shape while partial cannula (130) is semicircular. Cannula (120) and partial cannula (130) may be coextensive with their proximal end terminating within the valve assembly (200) and their distal end supporting tissue piercing tip (140). Although needle portion (110) is shown as having a generally ovular cross-section, it should be understood that other cross-sectional shapes may be used. Indeed, needle portion (110) may be comprised of only circular tubes thus creating a generally figure eight cross-section. Alternatively, needle portion (110) may be comprised of two square tubes thus creating a generally square cross-section. Yet in other configurations, needle portion (110) may be comprised of two concentric tubes thus creating a generally circular cross-section. In still other configurations, any other suitable shape may be used.

FIG. 6 depicts needle portion (110) with cutter (60) disposed therein. In particular, cannula (120) is configured to receive cutter (60) and to permit cutter (60) to translate and rotate within second lumen portion (162). Cannula (120) further comprises lateral aperture (150). Lateral aperture (150) is sized to receive prolapsed tissue during operation of biopsy device (10). The sidewall of cannula (120) opposite lateral aperture (150) comprises a plurality of openings (170) that provide fluid communication between first lumen portion (160) and second lumen portion (162). In the present example, first lumen portion (160) may selectively provide atmospheric air to vent second lumen portion (162) through the plurality of openings (170). Such an atmospheric vent in second lumen portion (162) allows severed tissue to be drawn through cutter (60) and into tissue sample holder (40) under the influence of vacuum from vacuum pump (50).

In use, cutter (60) can be moved through a variety of positions such as a closed position, an open position and finally in an intermediate position. Each position may correspond to a particular stage in the tissue sample extraction process. For example, the cannula (120) may penetrate a patient's tissue when cutter (60) is in a closed position. In the closed position, cutter (60) is in its furthest distal position relative to lateral aperture (150). Thus, cannula (120) may penetrate through tissue smoothly without catching any surrounding tissue that might impede penetration. In the open position, cutter (60) is in its furthest proximal position relative to lateral aperture (150). This state may, for example, correspond to a position where cannula (120) is oriented inside a patient where a tissue sample may be taken. With cutter (60) in its furthest proximal position relative to lateral aperture (150) a vacuum may be applied to prolapse patient's tissue through lateral aperture (150). Finally, when cutter (60) is in the intermediate position, cutter (60) is in a position between its furthest distal and proximal positions relative to lateral aperture (150). In this position, cutter (60) may be in a motive state from either a closed position or an open position to a closed or open position, respectively. For example, cutter (60) can move from an open to closed position so that cutter (60) may sever a tissue sample. Alternatively, cutter can move from a closed to open position in order to allow the patient's tissue to prolapse through lateral aperture (150). As will be described in further detail below, these various positions correspond to various pneumatic states of valve assembly (200). It should be understood that the various positions of cutter (60) and the corresponding stages in the tissue extraction process are merely exemplary and other suitable combinations will be apparent to one of ordinary skill in the art from the teachings herein.

Tissue piercing tip (140) is shown as having a generally conical body with a flat blade protruding therefrom. The shape of tissue piercing tip (140) is merely exemplary and many other suitable shapes may be used. For example, tissue piercing tip (140) may be in the shape of a blade protruding from needle portion (110), disregarding the conical body. Still in further variations, the tissue piercing tip (140) may have a flat blade portion of varying shapes and configurations. Other various configurations for tissue piercing tip (140) and for needle portion (110) in general may be provided as will be apparent to one of ordinary skill in the art in view of the teachings herein. By way of example only, needle portion (110) may be constructed in accordance with at least some of the teachings of U.S. Patent No. 8,801,742, entitled "Needle Assembly and Blade Assembly for Biopsy Device," issued August 8, 2014.

### C. Exemplary Valve Assembly

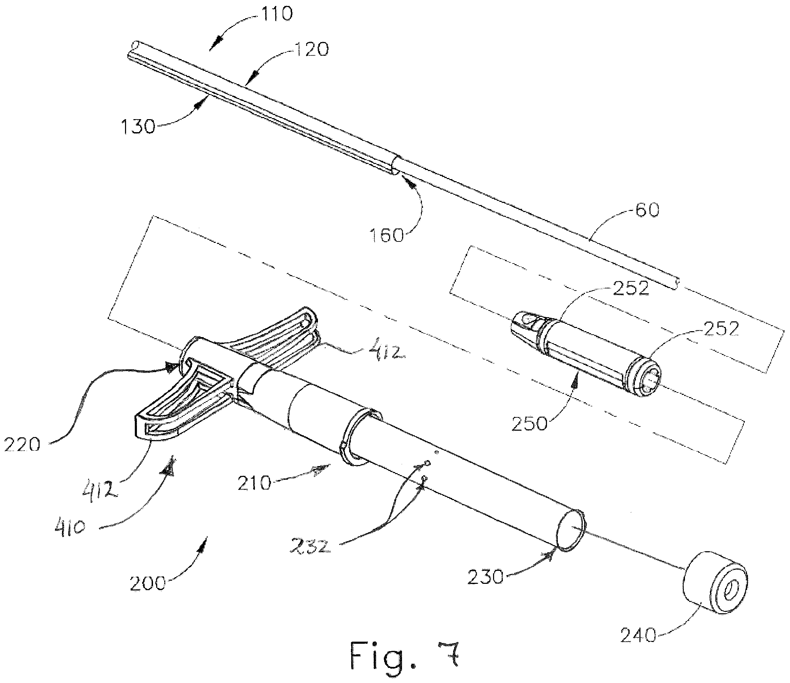

FIG. 7 depicts an exploded view of an exemplary valve assembly (200). Valve assembly (200) comprises a manifold (210), a static seal (240) and a spool body (250). Manifold (210) couples valve assembly (200) to the proximal end of needle portion (110) of needle assembly (100). In particular, manifold (210) comprises a needle coupling end (220) and a venting end (230). As is best seen in FIG. 7, needle coupling end (220) of manifold (210) is configured to receive the proximal end of needle portion (110) of needle assembly (100). In the present example, the coupling is made at the termination of cannula (120) and partial cannula (130). Cutter (60) then continues through valve assembly (200) to tissue sample holder (40). As will be described in more detail below, needle coupling end (220) creates an air tight seal around cannula (120) and partial cannula (130) to permit fluid flow from venting end (230) through first lumen portion (160). Coupling between needle portion (110) and needle coupling end (220) of manifold (210) may be facilitated by any suitable means such as adhesive bonding, a resilient sealing feature, an interference fitting, or a mechanical fastening means.

Venting end (230) extends proximally from needle coupling end (220). In the present example, needle coupling end (220) and venting end (230) are integrally formed as a single unit. In other examples, needle coupling end (220) and venting end (230) may be separate components joined together by any suitable fastening means. Venting end (230) terminates at the proximal end of manifold (210) where static seal (240) is affixed thereto. Venting end (230) defines a plurality of transverse openings (232) that are longitudinally co-located with each other. Transverse openings (232) are equidistantly spaced from each other about the outer perimeter of venting end (230) at their common longitudinal position. As will be described in greater detail below, transverse openings (232) provide communication of atmospheric air to the interior of venting end (230) such that atmospheric air can be fluidly communicated to first lumen portion (160).

Static seal (240) is affixed to the proximal end of manifold (210). Cutter (60) extends through static seal (240). Although cutter (60) is free to rotate and translate through static seal (240), static seal (240) prevents fluid communication at the interface between cutter (60) and static seal (240). Thus, with the seal created by static seal (240) and the seal created by needle coupling end (220), flow of atmospheric air can be limited to transverse openings (232) to first lumen portion (160).

Spool body (250) has o-rings (252) situated near the distal end and proximal end of spool body (250). As will be described in more detail below, o-rings (252) create a seal between spool body (250) and the inner diameter surface of venting end (230) of manifold (210). Although spool body (250) is shown with two o-rings (252), any suitable number of o-rings may be utilized. In some examples, spool body (250) can be connected directly to cutter (60) such that spool body (250) can more within manifold (210) as cutter (60) is moved. Additionally, in some examples spool body (250) can include one or more vent channels extending therethrough to promote fluid flow through spook body (250). By way of example only, spool body (250) may be constructed in accordance with at least some of the teachings of U.S. Pub. No. 2016/0287221, entitled "Biopsy Device with Translating Valve Assembly," published October 6, 2016.

In use, spool body (250) is moved within manifold (210) relative to vent openings (232) to change the pneumatic state of valve assembly (200). In such uses, movement of spool body (250) can be at least partially controlled by movement of cutter (60). For instance, in some examples, valve assembly (200) can be configured to vent second lumen (162) when cutter (60) is disposed in a distal position. In such a position, spool body (250) is driven distally such that 0-rings (252) are disposed distally of vent openings (232). Atmospheric air can then freely flow through vent openings (252) and spool body (250) into second lumen (162). Such a position can correspond to severing of a tissue sample using cutter (60). Thus, it should be understood that venting is provided to second lumen (162) after a tissue sample has been severed to promote tissue transport through cutter (60). In other examples, it may be desirable to substantially seal second lumen (162) relative to atmosphere. For instance, in the intermediate position described above, tissue may be prolapsed into lateral aperture (150). In this circumstance, it may be desirable to seal second lumen (162) to prevent escape of vacuum through vent openings (232). Accordingly, in this circumstance, spool body (250) can be positioned by cutter (60) such that 0-rings (252) are positioned on distally and proximally relative to vent openings (232). 0-rings (252) then seal second lumen (162) relative to atmosphere. Of course, various other additional or alternative pneumatic states can be used as will be apparent to those of ordinary skill in the art in view of the teachings herein. By way ofexample only, suitable pneumatic states can be in accordance with at least some of the teachings of U.S. Pub. No. 2016/0287221, entitled "Biopsy Device with Translating Valve Assembly," published October 6, 2016.

### D. Exemplary Needle Firing Assembly

In some examples it may be desirable to fire needle assembly (100) distally prior to collecting tissue samples. Although biopsy device (10) of the present example is generally configured for use in biopsy procedures that do not customarily use needle firing for insertion (e.g., ultrasonically guided procedures), in some uses operators may nonetheless prefer to have needle firing in at least some capacity. For instance, some operators may have a preference to quickly fire the needle through a targeted lesion after the needle has been inserted into a patient. Accordingly, in some examples it may be desirable to incorporate various needle firing features into biopsy device (10).

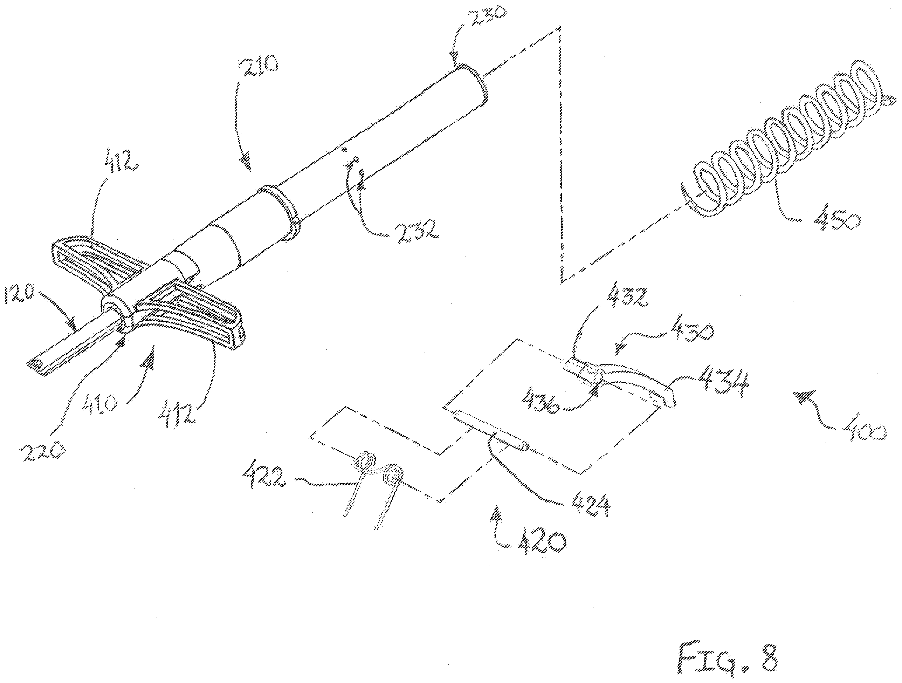

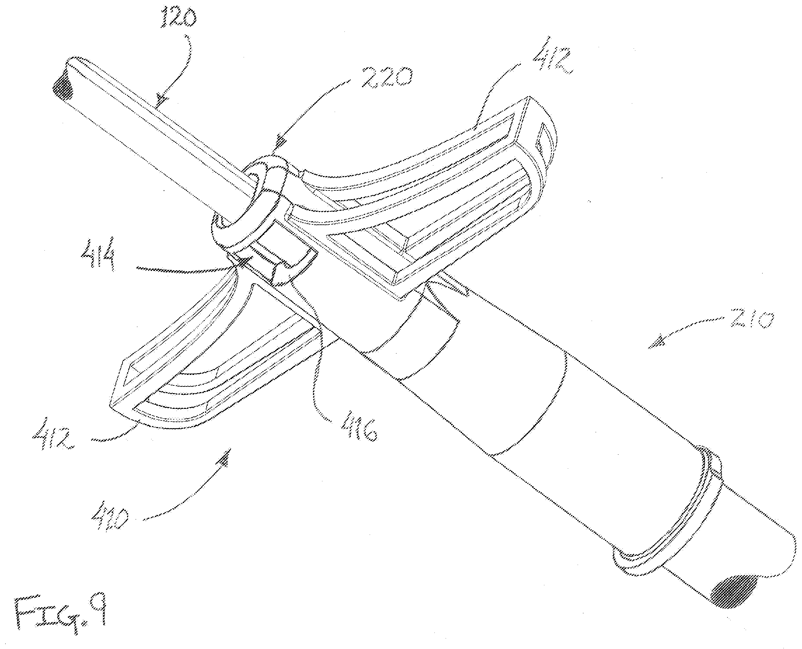

FIGS. 8 and 9 show an exemplary needle firing assembly (400). Needle firing assembly (400) is generally configured to manually cock and fire needle assembly (100) relative to probe housing (22, 24). As will be described in greater detail below, needle firing assembly (400) can be generally used to fire needle assembly (400) directly into a targeted lesion or mass of interest rather than requiring an operator to position needle assembly (100) underneath or around the lesion. Needle firing assembly (400) includes a cocking portion (410), a trip or release mechanism (420), and a resilient member or coil spring (450).

As best seen in FIGS. 8 and 9, cocking portion (410) includes two laterally extending arms (412) and a catch (414) (FIG. 9). In the present example, cocking portion (410) is generally integrated into the construction of manifold (210) of valve assembly (200). Since manifold (210) is fixedly secured to the proximal end of cannula (120) it should therefore be understood that movement or translation of manifold (210) by cocking portion (410) provides corresponding movement or translation of cannula (120). Although cocking portion (410) of the present example is shown as being integrated into manifold (210), it should be understood that in other examples cocking portion (410) can be configured as an entirely separate component attached to cannula (120) independently of manifold (210).

Arms (412) extend laterally from manifold (210) and generally protrude from probe housing (22, 24). As will be described in greater detail below, arms (412) are generally configured for grasping by an operator to pull manifold (210) and needle assembly (100) proximally to cock needle firing assembly (400). As such, it should be understood that arms (412) can include various grasping features. For instance, in the present example, arms (412) include a slight curvature to promote gasping. In other examples, arms can include ribs, and/or multiple curvatures to promote gasping. Arms (412) in the present example are also formed of a grid-like structure. Such a structure may prevent slippage by providing for fluid drainage. Of course, such a feature is merely optional and may be omitted in some examples in favor of solid arms (412).

As best seen in FIG. 9, catch (414) of the present example is defined by an opening in the underside of manifold (210) or by an indentation in the manifold. Catch (414) includes a distally facing wall (416). As will be described in greater detail below, wall (416) is generally configured to engage trip mechanism (420) to lock needle firing assembly (400) in a cocked position. As such, wall (416) provides a generally flat surface for one or more portions of trip mechanism to latch onto.

As best seen in FIG. 8, trip mechanism (420) includes a torsion spring (422), an anchor pin (424), and a release arm (430). Torsion spring generally fits around anchor pin (424) to bias release arm (430) around the axis of anchor pin (424) upwardly towards catch (414). Anchor pin (424) is configured to fasten to at least a portion of probe housing (22, 24) to permit pivoting of release arm (430) about an axis, as will be described in greater detail below.

Release arm (430) includes a lock tooth (432), a pivot arm (434) and pivot opening (436) disposed between lock tooth (432) and pivot arm (434). Lock tooth (432) is generally configured to engage wall (416) of catch (414), as will be described in greater detail below. Pivot arm (434) is generally configured to extend from the exterior of probe housing (22, 24) to permit an operator to pivot release arm (430) and thereby release, trigger or trip needle firing assembly (400). Pivot opening (436) is configured to receive anchor pin (434) to lock release arm (430) about a pivot axis while still permitting pivoting of release arm (430).

Coil spring (450) in the present example is used to bias cocking portion (410) towards a distal position. Although coil spring (450) is described herein as a coil spring, it should be understood that in other examples various alternative resilient mechanisms can be used such as a rubber band, an elastic chord or wire, or an elastic rod.

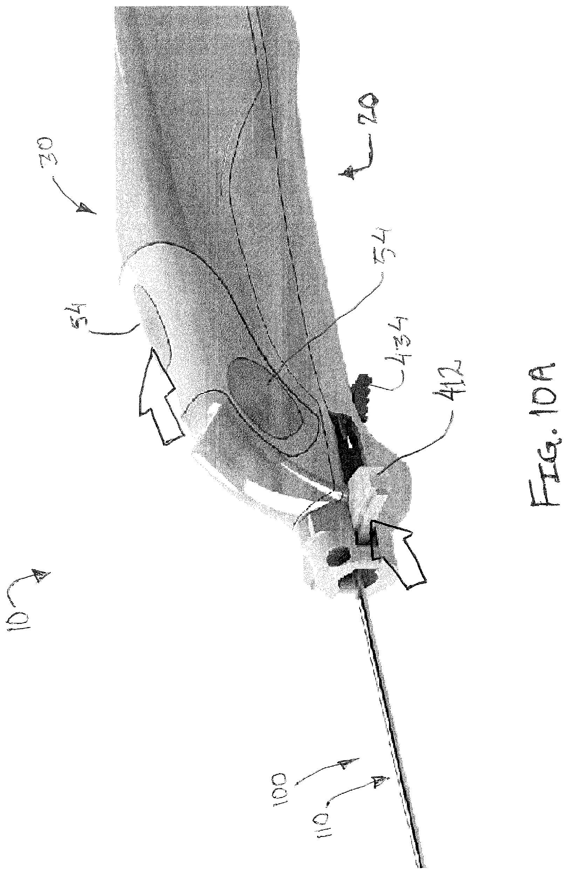

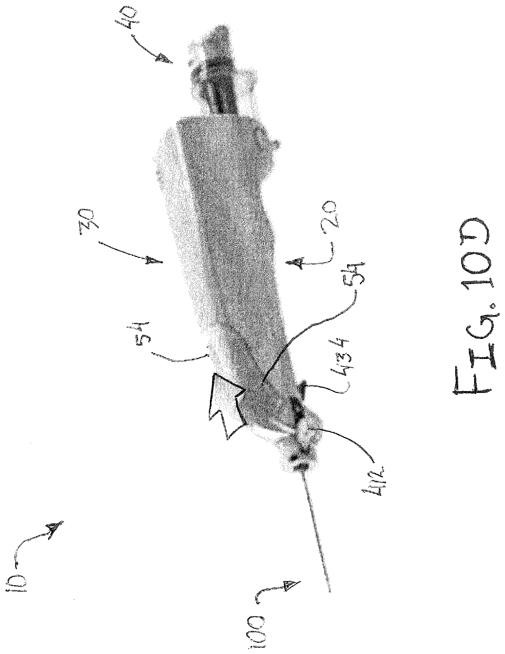

An exemplary use of needle firing assembly (400) is shown in FIGS. 10A through 11C. Prior to using needle firing assembly (400), biopsy device (10) can be initialized and prepared for a biopsy procedure. For instance, this process may begin by attaching holster (30) to probe (20). Control module (1000) within holster (30) will then begin an initialization sequence that can include, for example, closing cutter (60) completely, opening cutter (60) completely, then closing cutter (60) fully again.

Once initialization is complete, an operator may begin cocking needle firing assembly (400) for firing. As seen in FIG. 10A, an operator may first press one or more buttons (54) (as shown with arrow) to retract cutter (60) to the open position. At this stage, biopsy device (10) is prepared for the cocking procedure. The operator may then gasp arms (412) of cocking portion (410) and pull arms (412) and cocking portion (410) proximally against the resilient bias of coil spring (450). As shown in FIG. 10A, pulling arms (412) proximally results in corresponding proximal movement of needle assembly (100).

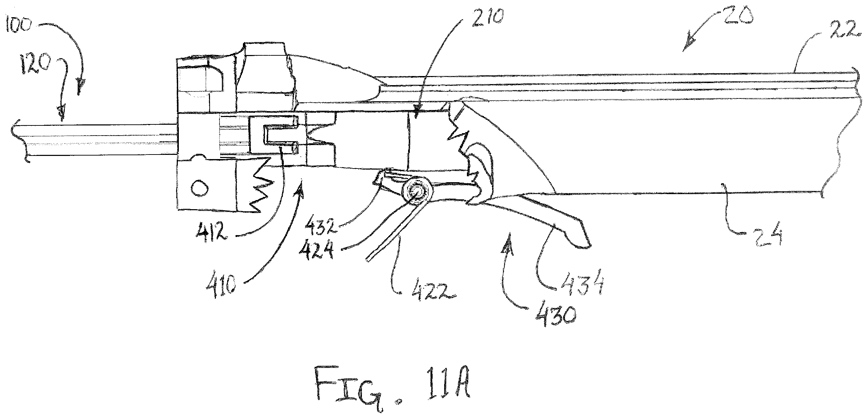

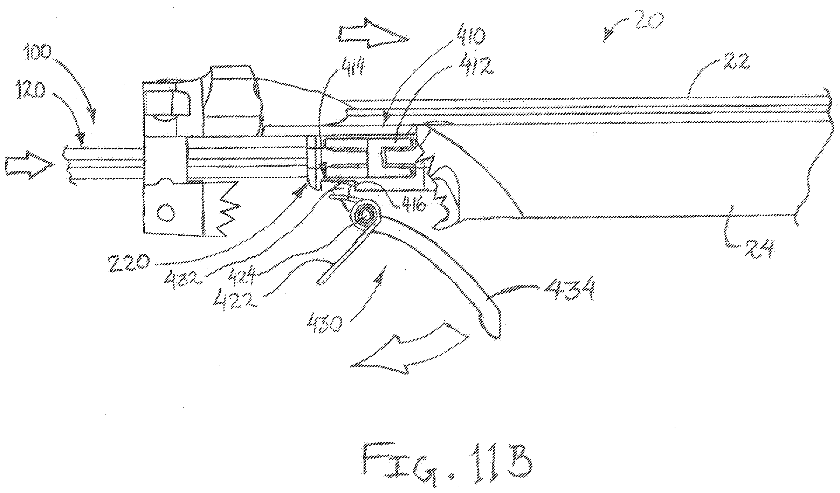

An operator can continue to pull arms (412) proximally until trip mechanism (420) engages catch (414) of cocking portion (410). In particular, as can be seen by comparing FIGS. 11A and 11B, proximal movement of cocking portion (410) results in catch (414) moving proximally relative to release arm (430). Once catch (414) is aligned with release arm (430) as shown in FIG. 11B, torsion spring (422) biases release arm (430) upwardly towards cocking portion (410) until lock tooth (432) engages wall (416) of catch (414). At this stage, an operator can release arms (412), and needle firing assembly (400) will remain in the cocked position until firing of needle assembly (100) is desired.

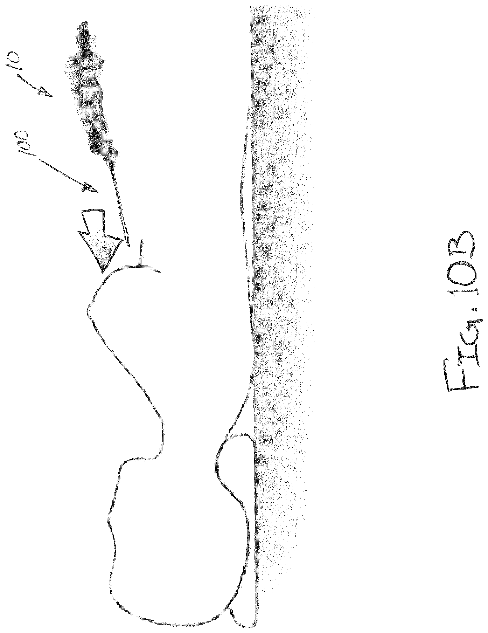

Once needle firing assembly (400) is cocked, an operator may insert a portion of needle assembly (100) into the patient as shown in FIG. 10B. Although not shown, it should be understood that prior to this stage the operator may also optionally push one or more buttons (54) to advance cutter (60) to the distal position. Such a use may be desirable to promote penetration of needle assembly (100) through tissue.

Regardless, once needle assembly (100) is inserted into the patient, an operator may position needle assembly (100) within the patient under image guidance (e.g., ultrasound) to position tissue piercing tip (140) adjacent to a target lesion or mass. Once tissue piercing tip (140) is positioned as desired, an operator may next desire to fire needle assembly (100) to thereby fire tissue piercing tip (140) through the target lesion or mass.

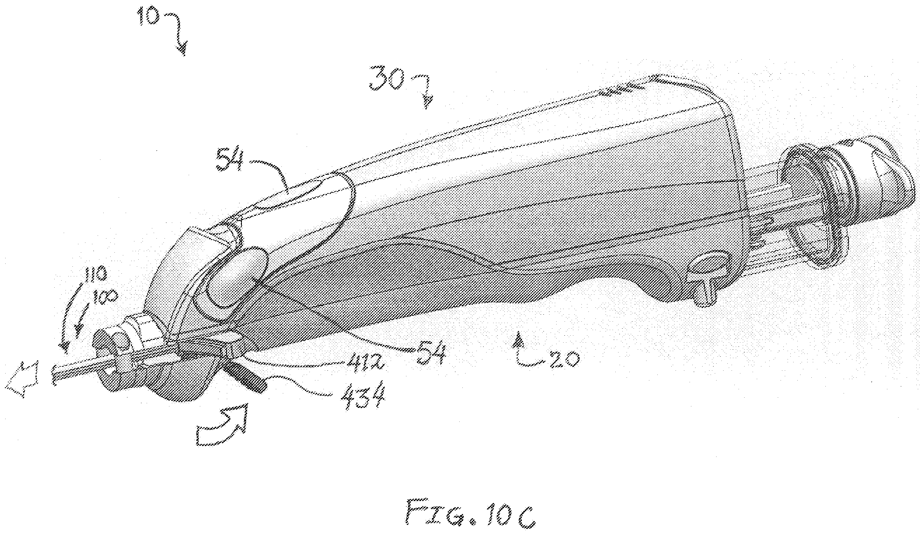

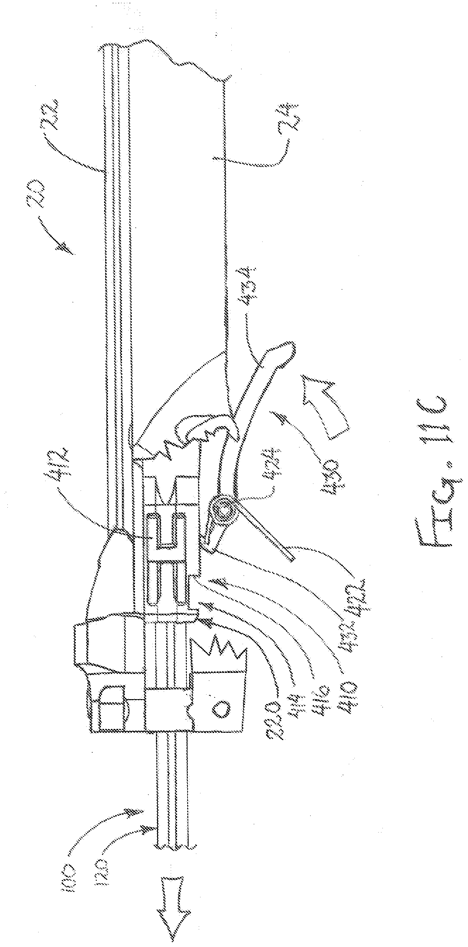

To fire needle assembly (100), an operator may pull pivot arm (434) of release arm (430) proximally and upwardly as shown in FIG. 10C. As shown in FIG. 11C, this movement pivots release arm (430) so that lock tooth (432) pivots away from wall (416) of catch (414) and thereby disengages therefrom. Once lock tooth (432) is disengaged from wall (416), the resilient bias of coil spring forces cocking portion (410) distally, which results in distal translation of needle assembly (100).

Once needle assembly (100) has been fired, tissue collection can be performed through lateral aperture (150) using cutter (60) by pressing one or more buttons (54). One or more tissue samples can be collected and deposited in tissue sample holder (40). Once a suitable number of tissue samples have been acquired, needle assembly (100) can be withdrawn from the patient. The tissue samples can then be sent to a pathology laboratory, probe (20) can be disposed of, and holster (30) can be cleaned and/or sterilized for subsequent procedures.

### IV. Exemplary Alternative Needle Firing Assembly

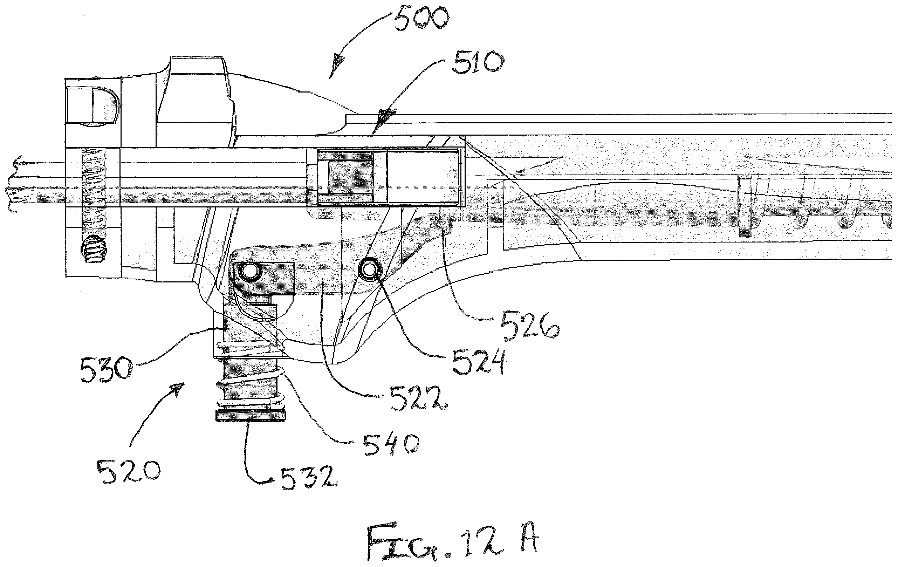

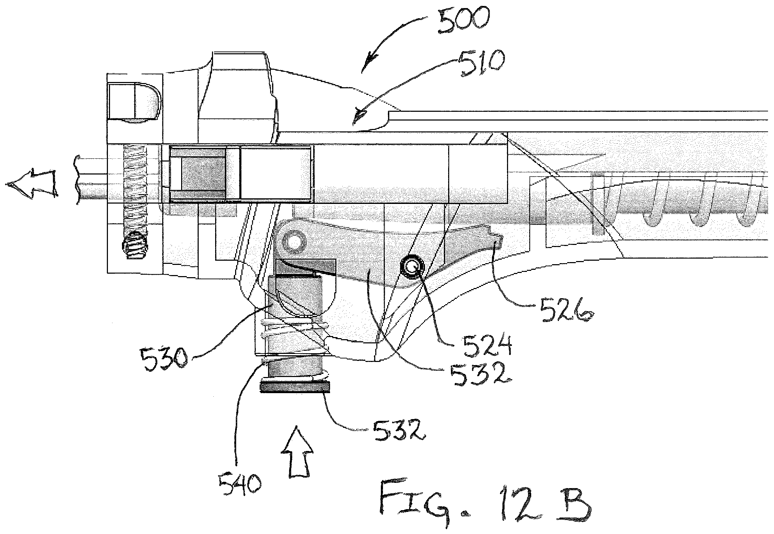

FIGS. 12A and 12B depict biopsy device (10) equipped with an exemplary alternative needle firing assembly (500). Needle firing assembly (500) is substantially similar to needle firing assembly (400) described above, unless otherwise explicitly noted herein. Additionally, it should be understood that needle firing assembly (500) operates substantially similarly as needle firing assembly (400) described above except as otherwise explicitly noted herein. For instance, needle firing assembly (500) includes a cocking portion (510) that is substantially similar to cocking portion (410) described above. However, unlike needle firing assembly (400) described above, needle firing assembly (500) of the present example uses a different trip mechanism (520). As can be seen, trip mechanism (520) generally includes a push button release mechanism. In particular, trip mechanism (520) includes a pivoting lever arm (522) that is pivotable about a pivot pin (524) to engage a lock tooth (526) with cocking portion (510).

A distal end of lever arm (522) is pivotably fastened to a push button (530). Push button (530) includes a flared lower end (532). A spring (540) is disposed between probe housing (22, 24) and lower end (532) to resiliently bias push button (530) laterally outwardly or downwardly. This resilient bias causes the distal end of lever arm (522) to also be biased downwardly such that lock tooth (526) is biased upwardly similarly to lock tooth (432) described above.

In use, needle firing assembly (500) is used substantially similarly to needle firing assembly (400) described above. For instance, like with needle firing assembly (400), needle firing assembly (500) is cocked by pulling cocking portion (510) proximally. This causes cocking portion (510) to engage cocking portion (510) once pulled proximally to a suitable distance via the resilient bias of lock tooth (526). However, unlike needle firing assembly (400), needle firing assembly (500) is fired by way of push button (530) rather than a lever mechanism as shown in FIG. 12B.

Embodiments of the devices disclosed herein can be designed to be disposed of after a single use, or they can be designed to be used multiple times. Embodiments may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, embodiments described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta or gamma radiation, ethylene oxide, or steam.

## Claims

1. A biopsy device, the biopsy device comprising:
(a) a probe (20);
(b) a needle (110) extending from the probe (20); and
(c) a cutter (60) disposed within the needle (110), wherein the cutter (60) defines a cutter lumen and at least partially defines a vent lumen (162) between an exterior of the cutter and an interior of the needle;
(d) a vent assembly (200) configured to transition the vent lumen between a vented state and a sealed state in response to movement of the cutter having a manifold (210) and a spool body (250) movable within the manifold (210); and
(e) a manually actuated needle firing assembly (400) including a catch (414) and a release (420), wherein the catch (414) is resiliently biased to fire the needle (110), wherein the release (420) is configured to engage the catch (414) to thereby hold the catch (414) in a cocked position,
**characterized in that**
the catch (414) is integral with the manifold (210) which is coaxial with the needle (110), the catch (414) being defined by an opening in the underside of the manifold (210) or by an indentation in the manifold (210), wherein the indentation defines a distally facing wall.

2. The biopsy device of claim 1, wherein the release (420) includes a pivotable arm (430) configured to pivot into and out of engagement with the catch (414).

3. The biopsy device of claim 1, wherein the release includes a push button (530) configured to engage and disengage a lock tooth (526) with the catch (414).

4. The biopsy device of claim 1, wherein the release (420) is configured to engage the distally facing wall (416) to selectively lock the needle firing assembly (400) in the cocked position.

5. The biopsy device of any one or more of claims 1 through 4, further comprising a cutter drive assembly (300), wherein the cutter drive assembly includes a first gear (310) and a second gear (312) coaxial with the cutter (60), wherein the first gear (310) and second gear (312) are configured to rotate at different rotational speeds simultaneously to translate and rotate the cutter.

6. The biopsy device of any one or more of claims 1 through 5, further comprising a tissue sample holder (40) in communication with the cutter (60), wherein the tissue sample holder (40) is configured to collect a plurality of tissue samples within a chamber defined therein.

## Patentansprüche

1. Biopsievorrichtung, wobei die Biopsievorrichtung umfasst:
a) eine Sonde (20);
(b) eine Nadel (110), die sich von der Sonde (20) aus erstreckt; und
(c) ein innerhalb der Nadel (110) angeordnetes Schneidewerkzeug (60), wobei das Schneidewerkzeug (60) ein Schneidewerkzeuglumen definiert und zumindest teilweise ein Entlüftungslumen (162) zwischen einer Außenseite des Schneidewerkzeugs und einer Innenseite der Nadel definiert;
(d) eine Entlüftungsanordnung (200), die so konfiguriert ist, dass sie das Entlüftungslumen als Reaktion auf eine Bewegung des Schneidewerkzeugs zwischen einem entlüfteten Zustand und einem abgedichteten Zustand überführt, die einen Verteiler (210) und einen Spulenkörper (250) aufweist, der innerhalb des Verteilers (210) beweglich ist; und
(e) eine manuell betätigte Nadelabschussanordnung (400), einschließlich einer Klinke (414) und eines Auslösers (420), wobei die Klinke (414) elastisch vorgespannt ist, um die Nadel (110) abzufeuern, wobei der Auslöser (420) so konfiguriert ist, dass er in die Klinke (414) eingreift, um dadurch die Klinke (414) in einer gespannten Stellung zu halten,
**dadurch gekennzeichnet, dass**
die Klinke (414) einstückig mit dem Verteiler (210) ausgebildet ist, der koaxial mit der Nadel (110) angeordnet ist, wobei die Klinke (414) durch eine Öffnung in der Unterseite des Verteilers (210) oder durch eine Vertiefung im Verteiler (210) definiert ist, wobei die Vertiefung eine distal ausgerichtete Wand definiert.

2. Biopsievorrichtung nach Anspruch 1, wobei der Auslöser (420) einen schwenkbaren Arm (430) einschließt, der so konfiguriert ist, dass er in Eingriff mit der Klinke (414) und aus diesem heraus schwenkt.

3. Biopsievorrichtung nach Anspruch 1, wobei der Auslöser einen Druckknopf (530) einschließt, der dazu konfiguriert ist, einen Sperrzahn (526) mit der Klinke (414) in Eingriff zu bringen und von dieser zu lösen.

4. Biopsievorrichtung nach Anspruch 1, wobei der Auslöser (420) so konfiguriert ist, dass er in die distal ausgerichtete Wand (416) eingreift, um die Nadelabschussanordnung (400) in der gespannten Position selektiv zu verriegeln.

5. Biopsievorrichtung nach einem oder mehreren der Ansprüche 1 bis 4, weiter umfassend eine Schneidwerkzeug-Antriebsanordnung (300), wobei die Schneidwerkzeug-Antriebsanordnung ein erstes Zahnrad (310) und ein zweites Zahnrad (312) einschließt, die koaxial mit dem Schneidewerkzeug (60) angeordnet sind, wobei das erste Zahnrad (310) und das zweite Zahnrad (312) so konfiguriert sind, dass sie sich gleichzeitig mit unterschiedlichen Drehgeschwindigkeiten drehen, um das Schneidewerkzeug zu verschieben und zu drehen.

6. Biopsievorrichtung nach einem oder mehreren der Ansprüche 1 bis 5, weiter umfassend einen Gewebeprobenhalter (40), der mit dem Schneidewerkzeug (60) in Verbindung steht, wobei der Gewebeprobenhalter (40) so konfiguriert ist, dass er innerhalb einer darin definierten Kammer eine Vielzahl von Gewebeproben sammelt.

## Revendications

1. Dispositif de biopsie, le dispositif de biopsie comprenant :
(a) une sonde (20) ;
(b) une aiguille (110) s'étendant à partir de la sonde (20) ; et
(c) un moyen de coupe (60) disposé à l'intérieur de l'aiguille (110), dans lequel le moyen de coupe (60) définit une lumière de moyen de coupe et définit au moins partiellement une lumière d'évent (162) entre un extérieur du moyen de coupe et un intérieur de l'aiguille ;
(d) un ensemble d'évent (200) configuré pour faire la transition de la lumière d'évent entre un état ventilé et un état étanche en réponse à un mouvement du moyen de coupe présentant un collecteur (210) et un corps de bobine (250) mobile à l'intérieur du collecteur (210) ; et
(e) un ensemble de déclenchement d'aiguille (400) à actionnement manuel incluant un moyen d'arrêt (414) et un moyen de libération (420), dans lequel le moyen d'arrêt (414) est sollicité de manière résiliente pour déclencher l'aiguille (110), dans lequel le moyen de libération (420) est configuré pour venir en prise avec le moyen d'arrêt (414) pour ainsi retenir le moyen d'arrêt (414) dans une position armée,
**caractérisé en ce que**
le moyen d'arrêt (414) fait partie d'un seul tenant avec le collecteur (210) qui est coaxial avec l'aiguille (110), le moyen d'arrêt (414) étant défini par une ouverture dans le dessous du collecteur (210) ou par une indentation dans le collecteur (210), dans lequel l'indentation définit une paroi en regard distalement.

2. Dispositif de biopsie selon la revendication 1, dans lequel le moyen de libération (420) inclut un bras pivotant (430) configuré pour pivoter en et hors mise en prise avec le moyen d'arrêt (414).

3. Dispositif de biopsie selon la revendication 1, dans lequel le moyen de libération inclut un bouton poussoir (530) configuré pour venir en prise avec et se désengager d'une dent de verrouillage (526) avec le moyen d'arrêt (414).

4. Dispositif de biopsie selon la revendication 1, dans lequel le moyen de libération (420) est configuré pour venir en prise avec la paroi en regard distalement (416) afin de verrouiller sélectivement l'ensemble de déclenchement d'aiguille (400) dans la position armée.

5. Dispositif de biopsie selon l'une quelconque ou plus des revendications 1 à 4, comprenant en outre un ensemble d'entraînement de moyen de coupe (300), dans lequel l'ensemble d'entraînement de moyen de coupe inclut un premier engrenage (310) et un second engrenage (312) de manière coaxiale avec le moyen de coupe (60), dans lequel le premier engrenage (310) et le second engrenage (312) sont configurés pour tourner à différentes vitesses de rotation de manière simultanée afin de translater et faire tourner le moyen de coupe.

6. Dispositif de biopsie selon l'une quelconque ou plus des revendications 1 à 5, comprenant en outre un moyen de retenue d'échantillon de tissu (40) en communication avec le moyen de coupe (60), dans lequel le moyen de retenue d'échantillon de tissu (40) est configuré pour collecter une pluralité d'échantillons de tissu au sein d'une chambre définie à l'intérieur.
